# EUROPÄISCHE PATENTSCHRIFT

(11) **EP 0 625 354 B1**
(45) Veröffentlichungstag und Bekanntmachung des Hinweises auf die Patenterteilung: **13.12.2000**
(21) Anmeldenummer: 94105595.6
(22) Anmeldetag: 12.04.1994
(51) Int. Cl.: A61K 38/00

(54) **Arzneimittel enthaltend die Untereinheit p40 von Interleukin-12**
Medical preparation containing the subunit p40 of interleukin 12
Préparation médicinale contenant l'unité subordonnée p40 de interleucine 12

(30) Priorität: 07.05.1993 DE 4315127
(43) Veröffentlichungstag der Anmeldung: 23.11.1994
(73) Patentinhaber: HOECHST AKTIENGESELLSCHAFT, 65926 Frankfurt am Main (DE)
(72) Erfinder: Seiler, Friedrich-Robert, Dr., D-35041 Marburg (DE); Kurrle, Roland, Dr., D-35096 Niederweimar (DE); Langner, Klaus-Dieter, Dr., D-35041 Marburg (DE)
(74) Vertreter: Fischer, Hans-Jürgen, Dr.

(56) Entgegenhaltungen:
- ARCHIVES OF BIOCHEMISTRY AND BIOPHYSICS, Band 294, Nr. 1, April 1992 F.J. PODLASKI et al. "Mole- cular Characterization of Interleukin 12" Seiten 230-237

## Beschreibung

Die vorliegende Erfindung betrifft die Verwendung der Untereinheit p40 von Interleukin-12 zur Herstellung eines Arzneimittels zur Behandlung von Krankheiten, die mit einer Fehlregulation des Immunsystems nämlich einer Dysregulation IL-12 vermittelter Aktivitäten, einhergehen.

Immunreaktionen können durch unterschiedliche T-Zell Populationen unterstützt werden. Je nach Typ der Immunreaktion leisten dann bevorzugt T-Helfer Zellen des Typ 1 (TH1) oder T-Helfer Zellen des Typ 2 (TH2) Hilfe für eine Immunreaktion. TH1-Zellen unterscheiden sich von TH2-Zellen, soweit bisher bekannt, insbesondere durch die Produktion unterschiedlicher Cytokine.

Bereits 1989 konnten Dunn et al. (J. Immunol. 142:3847 ff, 1989) zeigen, daß TH-1 Klone gamma-IFN produzieren, vorausgesetzt, sie wurden in Anwesenheit akzessorischer Zellen und IL-2 kultiviert. Außerdem konnten Germann et al. (Eur. J. Immunol., 8:1857-1862, 1991) zeigen, daß für die gamma-IFN-Synthese durch TH1 Zellen ein löslicher Mediator benötigt wird. Aus vergleichenden Untersuchungen ging hervor, daß der von Germann et al. als TSF bezeichnete Mediator identisch mit murinem IL-12 ist. Kürzlich konnte dann von Mengel et al. (Eur. J. Immunol. 22:3173-3178, 1992) im Überstand der aktivierten murinen B-Zell Lymphomalinie A-20 ebenfalls ein löslicher Faktor beschrieben werden, der die gamma-IFN Produktion bei T-Zellen stimuliert. Der im A-20 Überstand von Mengel beschriebene lösliche Mediator wird funktionell mit einem sogenannten "Human Natural Killer Cell Stimulatory Faktor (NKSF)" (Wolf, S.F. et al., J. Immunol., 156:3074, 1991) verglichen und als murines Analog zum humanen IL-12 postuliert. Sowohl von IL-12 als auch von NKSF, die potentiell identisch sind, ist bekannt, daß sie die gamma-IFN Synthese stimulieren. Selbstverständlich ist die Funktion von IL-12 (entsprechend NKSF) nicht auf die Stimulation der gamma-IFN Produktion beschrankt. Unter anderem beeinflußt IL-12 (entsprechend NKSF) auch die Funktion von natürlichen Killer-Zellen (sogenannten NK- oder LGL-Zellen), die IgE-Produktion und steigert die IL-12 induzierte Proliferation ruhender peripherer mononuclearer Zellen.

Von IL-12 ist außerdem bekannt, daß es aus zwei verschiedenen Untereinheiten besteht, die als p35 und p40 bezeichnet werden (Podlaski, F. J. et al. (1991) Arch. Biochem. Biophys. Vol. 294, No. 1, pp. 230-237). Eine nahezu identische Struktur wurde auch für murines IL-12 gefunden (Schoenhaut D. S. et al. (1992) J. Immunol. Vol. 148, No. 11, pp. 3433-3440). Für die p40 Untereinheit von IL-12 wurde gezeigt, daß sie alleine zwar keine IL-12-spezifische Bioaktivität besitzt, daß sie aber für die Bioaktivität des kompletten IL-12-Moleküls von wesentlicher Bedeutung ist. Es wurde spekuliert, daß die p40-Untereinheit direkt mit dem zellulären IL-12-Rezeptor interagiert.

Die Fehlregulation der Produktion und der Wirkung von Cytokinen ist gemäß einer ganzen Reihe von Publikationen an der Entstehung akuter und chronischer Erkrankungen des Immunsystems mit ihren Folgeerscheinungen ursächlich beteiligt. Ein therapeutischer Ansatz könnte daher in der Beeinflußung cytokinvermittelter Aktivitäten liegen.

Der vorliegenden Erfindung liegt daher die Aufgabe zu Grunde, ein Arzneimittel zur Verfügung zu stellen, das sich vorteilhaft zur Behandlung pathologischer Zustände einsetzen läßt, die mit einer Dysregulation IL-12-vermittelter Aktivitäten einhergehen.

Überraschenderweise wurde nun gefunden, daß sich die p40-Untereinheit des IL-12 zur Inhibition IL-12-vermittelter Aktivitäten einsetzen läßt. Damit ist es nun möglich, durch ein Eingreifen in die genannten Regulationsmechanismen eine therapeutische Wirkung zu erzielen.

Die vorliegende Erfindung betrifft somit die Verwendung einer natürlichen oder rekombinanten, möglicherweiser modifizierten Untereinheit p40 von Il-12 aus Säugern. Modifizierte Untereinheiten p40 im Sinne der Erfindung sind wirksame Teile oder Varianten von p40, wobei Wirksamkeit beispielsweise im Tiermodell (Maus) oder in vitro, entsprechend den in den Beispielen angegebenen Verfahren nachgewiesen werden kann.

Eine bevorzugte Ausführungsform der Erfindung betrifft die Verwendung einer Untereinheit p40 humanen Ursprungs.

Ein weiterer Aspekt der Erfindung betrifft die Verwendung einer natürlichen oder rekombinanten Untereinheit p40 von Il-12 aus Säugern, bevorzugt aus Geweben oder Zellen humanen Ursprungs zur Herstellung eines Diagnostikums zum Nachweis von IL-12.

Die vorliegende Erfindung betrifft weiterhin die Verwendung der natürlichen oder rekombinanten, möglicherweise modifizierten Untereinheit p40 von Il-12 aus Säugern, bevorzugt menschlichen Ursprungs zur Herstellung eines Arzneimittels zur Behandlung pathologischer Zustände, die mit einer Dysregulation Il-12-vermittelter Aktivitäten einhergehen, wie z. B. Autoimmunerkrankungen, wie Systemischer Lupus erythematodes, Wegeners Syndrom oder rheumatische Arthritis, bakterielle oder virale Infektionen und bestimmte solide Tumoren oder Leukämien.

Ein weiterer Aspekt der Erfindung betrifft die Verwendung der natürlichen oder rekombinanten, möglicherweise modifizierten Il-12-Untereinheit p40 aus Säugern in einem Verfahren zum Nachweis von Il-12 in Körperflüssigkeiten, z. B. vom Menschen. Dieses Verfahren beruht auf einer spezifischen Inhibition der Il-12-Aktivität durch p40 und kann z. B. analog zu den Beispielen 1 bzw. 2 erfolgen, indem das eingesetzte Il-12 aus der zu analysierenden Probe stammt und p40 in einer definierten Menge zugesetzt wird.

Bevorzugt sind alle diejenigen der genannten Verfahren, die natürliches oder rekombinantes p40 humanen Ursprungs verwenden.

Ein weiterer Aspekt der Erfindung betrifft ein Diagnostikum enthaltend Il-12 zum Nachweis der Il-12-Untereinheit p40. Die Anwendung dieses diagnostischen Mittels kann z. B. analog zu den Beispielen 1 bzw. 2 erfolgen, indem die eingesetzte p40-Inhibitoraktivität aus der zu analysierenden Probe stammt und Il-12 in einer definierten Menge zugesetzt wird.

Die Erfindung wird außerdem durch die Beispiele und die Ansprüche erläutert.

### Gewinnung von IL-12 und p40:

Zur Gewinnung von rekombinantem murinen IL-12 bzw. der p40 Untereinheit von mIL-12 wurde über Standardverfahren (Sambrook, J. Fritsch, E., Maniatis, T., (1989) Molecular Cloning: A Laboratory Manual, Cold Spring Harbor Laboratory Press, Gold Spring Harbor, N.Y.) aus murinen Milzzellen mRNA isoliert und diese in doppelsträngige cDNA überführt.

Unter Verwendung der in Schoenhaut et al. (Schoenhaut, D., Chua, A.O., Wolitzky, A.G., Quinn, P.M., Dwyer, C.M., McComas, W., Familletti, P.C. Gately, M.K., Gubler, U. (1992). J. Immunol. 148, 3433) beschriebenen Primer und unter den vorgegebenen experimentellen Bedingungen wurde eine PCR durchgeführt, bei der ein etwa 800 Basenpaar-Fragment generiert werden konnte.

Das PCR-Fragment wurde nach Standardverfahren sequenziert (Sambrook J. Fritsch, E., Maniatis, T., (1989) Molecular Cloning: A Laboratory Manual, Gold Spring Harbor Laboratory Press, Gold Spring Harbor, N.Y.). Als Ergebnis wurde gefunden, daß die experimentel ermittelte cDNA-Sequenz identisch mit der publizierten Sequenz der murinen p40-Untereinheit ist. In analoger Weise wurde das PCR-Fragment für die p35-Untereinheit isoliert und sequenziert. Zur Bestätigung, daß die nachfolgend beschriebenen biologischen Aktivitäten tatsächlich von IL-12 bzw. von der p40-Untereinheit resultieren, wurden die PCR-Fragmente einzeln bzw. kombiniert in den Vektor pABstop einkloniert und über ein Standardverfahren in BHK-21 Zellen stabil exprimiert (Zettlmeißl G, Wirth, M., Hauser, G., Küpper, H.A. (1988) Behring Inst. Mitteilungen 82, 26).

Aus Kulturüberständen von transfizierten BHK-21 Zellen konnte sowohl biologisch aktives mIL-12 bzw. eine biologisch aktive p40-Untereinheit von mIL-12 isoliert werden.

Als Quelle für ein natürliches IL-12 dienten Überstände der murinen B-Zell-Lymphomalinie A-20 (American Type Culture Collection, ATCC TIB208), die entsprechend der von Mengel et al. (s.o.) beschriebenen Methode aktiviert wurde. Der im A-20 Überstand von Mengel beschriebene lösliche Mediator wird funktionell mit NKSF verglichen und gilt als murines Analog zum humanen IL-12. Eine weitere Quelle für natürliches IL-12 stellten Milzzellpräparationen dar, die entsprechend der von Germann et al. (s.o.) beschriebenen Methode hergestellt und aktiviert wurden. Vergleichende Untersuchungen zeigten, daß das von Germann et al. beschriebene TSF identisch mit mIL-12 ist.

Als natürliche Quelle für die p40-Untereinheit des murinen IL-12 konnte ein Hybridom isoliert werden, das die p40-Untereinheit des murinen IL-12 sezerniert. Zur Herstellung dieses Hybridoms wurden weibliche Ratten (Stamm Lewis, Zentralinstitut für Versuchstierkunde, Hannover) mit 1-10x10⁶ murinen T-Zellen, die in Anwesenheit von syngenen Monozyten (1x10⁵/ml) und rekombinantem murinem GM-CSF (50 ng/ml) kultiviert worden waren, zusammen mit CFA (Complete Freund's adjuvants) subkutan injiziert. Zwei weitere Immunisierungen erfolgten im Abstand von jeweils 2 Wochen, wobei gleiche Zellmengen intraperitoneal injiziert wurden. 3 Tage nach der letzten Injektion wurden die Tiere getötet und die Milzzellen nach dem allseits bekannten Standardverfahren von Köhler und Milstein (Nature 256, p. 495ff; 1975) mit den Zellen der murinen Myeloma-Zellinie SP2/F0 fusioniert. Die Selektion der auswachsenden Hybridoma erfolgte nach Standardverfahren. Untersuchungen, die analog zu dem in Beispiel 1 dargestellten Experiment durchgeführt wurden, zeigten, daß Überstände eines der isolierten Hybridome eine inhibitorische Aktivität auf die gamma-Interferon Freisetzung besitzt. Aus dem Kulturüberstand dieses Hybridoms wurde in Anlehnung an das publizierte Verfahren von Kobayashi et al. ein sezerniertes Protein gereinigt (Kobayashi, M.; Fritz, L., Ryan, M., Hewick, R.M., Clar, S.C., Chan, S., Loudon, R., Sherman, F., Perussia, B., Trinchieri, G. (1989) J. Exp. Med. 170, 827). Nach Reinigung über reverse phase HPLC wurde die isolierte Proteinfraktion in einer SDS-Page aufgetrennt, es wurde eine dominante Proteinbande im Bereich von etwa 40-45 kDda gefunden. Sequenzvergleiche bestätigten, daß das hier isolierte Protein identisch mit der p40 Untereinheit des murinen IL-12 ist.

Das Arzneimittel wird schließlich nach dem Fachmann an sich bekanntem Verfahren hergestellt. Die Il-12-Untereinheit p40 (= der Wirkstoff) wird entweder als solche oder in Kombination mit geeigneten pharmazeutischen Zusatz- oder Hilfsstoffen sowie physiologisch annehmbaren Lösungsmitteln in einer wirksamen Konzentration eingesetzt.

### Beispiel 1:

### Inhibition der IL-12 induzierten gamma-IFN Freisetzung durch p40/IL-12

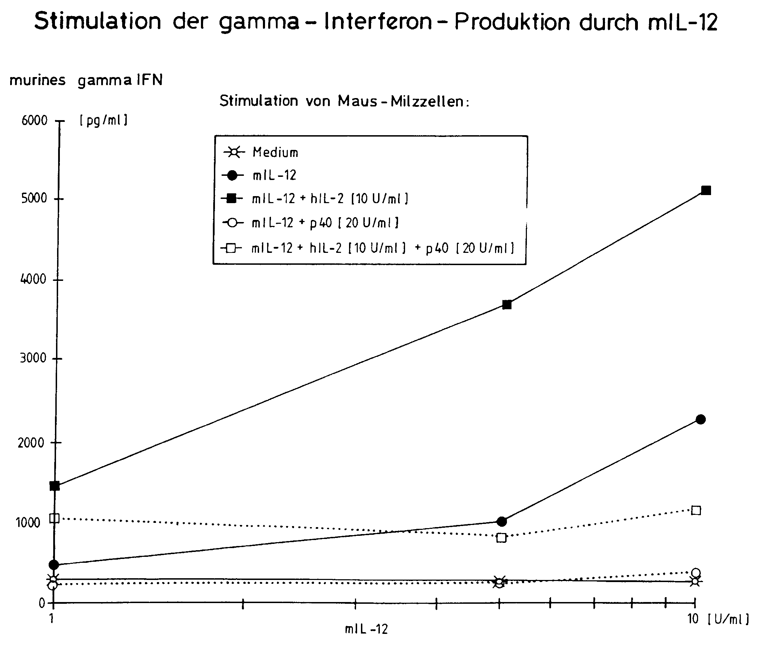

Zur Induktion der gamma-IFN Freisetzung wurden 5x10⁶ Milzzellen von BALB/c-Mäusen für 48 Std. in Anwesenheit bzw. Abwesenheit von rekombinantem oder natürlichem mIL-12 und IL-2 in den jeweils angegebenen Konzentrationen kultiviert. Nach 48 Std. Kultur wurde der Überstand der Milzzellen geerntet und dieser zellfrei zentrifugiert. Der gamma-IFN-Gehalt des Überstands wurde in kommerziell erhältlichen ELISA-Systemen (z.B. Intertest™-gamma, Mouse IFN-gamma ELISA-kit, Genzyme) bestimmt. Die Quantifizierung von gamma-IFN aus dem Kulturüberstand aktivierter Milzzellen erfolgte im Vergleich zu rekombinantem murinen gamma-IFN (Genzyme). Ein typisches Experiment ist in der Abbildung dargestellt. Wie die Daten zeigen, führte die Kultivierung von murinen Milzzellen mit IL-12 dosisabhängig zu einer gamma-Interferon-Freisetzung von > 5 ng/ml. Wurden jedoch die Milzzellen bei Kulturbeginn mit rekombinantem murinen p40/IL-12 oder mit Hybridoma-Überstand, der die natürliche p40 Untereinheit von mIL-12 enthielt vorinkubiert, und anschließend mit mIL-12 stimuliert, so wurde die IL-12 abhängige gamma-IFN Synthese um mindestens 50 % inhibiert. Dieses hier vorgestellte Beispiel zeigt, daß sowohl rekombinantes p40/IL-12 als auch Hybridoma-Überstand, der die natürliche p40 Untereinheit von mIL-12 enthält, in der Lage ist, die IL-12 induzierte gamma-IFN Synthese zu inhibieren.

### Beispiel 2:

### Inhibition der IL-12 induzierten NK-Zellaktivität durch p40/IL-12

Milzzellen von C57BL6 Mäusen wurden in einer Zelldichte von 5-10x10⁶ Zellen/ml in 24-Loch Costar-Platten für 18 Std. bei 37°C in serumfreiem Iscove's Medium kultiviert. Die Kultivierung der Milzzellen erfolgte in unterschiedlichen Konzentrationen von rekombinantem oder natürlichem murinen IL-12. Nach 18 Std. wurden die Zellen geerntet, die Zellzahl der lebenden Zellen durch Trypan-blau Färbung bestimmt und die zytolytische Aktivität in einem 5-stündigen ⁵¹Cr-Freisetzungstest bestimmt. Als Zielzellen dienten YAC-1 Zellen (ATCC TIB160). Die ⁵¹Cr-Markierung der Zielzellen und die Testdurchführung erfolgte nach Standardmethoden (z. B. Schoenhaut D.S. et al. (1992) J. Immunol. Vol. 148, No. 11, pp. 3433-3440). Die Verhältnisse von Effektor- zu Zielzellen betrugen typischerweise 100:1, 50:1, 25:1, 12.5:1. Die %-spezifische Zytolyse wurde kalkuliert als (%-Lyse der Experimentalgruppe - % Spontanlyse) : (% Maximallyse - % Spontanlyse) x 100. Durch Vorinkubation von C57BL/6-Milzzellen mit IL-12 konnte die spezifische Zytolyse gegenüber Ausgangskontrolle (Ratio 50:1) um mindestens das 5fache gesteigert werden. Wurden unter gleichen Kulturbedingungen die Milzzellen jedoch zusätzlich mit rekombinantem oder natürlichem murinen p40/IL-12 vorinkubiert, so wurde die IL-12 abhängige Zytolyse wiederum um mindestens 50% inhibiert.

## Patentansprüche

1. Verwendung der natürlichen oder rekombinanten Untereinheit p40 von IL-12 aus Säugern oder Teile oder Varianten der Untereinheit p40 von IL-12 aus Säugern, die IL-12-vermittelte Aktivität inhibieren, zur Herstellung eines Arzneimittels zur Behandlung von pathologischen Zuständen, die mit einer Dysregulation IL-12-vermittelter Aktivitäten einhergehen.

2. Verwendung gemäß Anspruch 1, dadurch gekennzeichnet, daß die p40 Untereinheit von lL-12 humanen Ursprungs ist.

3. Verwendung gemäß Anspruch 1, dadurch gekennzeichnet, daß es sich bei den pathologischen Zuständen um Autoimmunerkrankungen wie Systemischer Lupus erythematodes, Wegners Syndrom oder rheumatische Arthritis, bakterielle oder virale Infektionen oder um bestimmte solide Tumore oder Leukämie handelt.

4. Verwendung der natürlichen oder rekombinanten Untereinheit p40 von IL-12 aus Säugern oder Teile oder Varianten der Untereinheit p40 von IL-12 aus Säugern, die IL-12-vermittelte Aktivität inhibieren, zur Herstellung eines Diagnostikums zum Nachweis von IL-12.

5. Verwendung gemäß Anspruch 4, dadurch gekennzeichnet, daß die p40 Untereinheit von IL-12 humanen Ursprungs ist.

6. Verfahren zum Nachweis der Untereinheit p40 von IL-12 aus Säugern, dadurch gekennzeichnet, daß eine zu analysierende Probe entnommen wird, die die Untereinheit p40 von IL-12 aus Säugern enthält, eine definierte Menge IL-12 zugesetzt und die Inhibitoraktivität von p40 auf IL-12 gemessen wird.

7. Verfahren gemäß Anspruch 6, dadurch gekennzeichnet, daß die zu analysierende Probe aus einer menschlichen Körperflüssigkeit stammt.

8. Diagnostikum, enthaltend die natürliche oder rekombinante Untereinheit p40 von IL-12 aus Säugern oder Teile oder Varianten der Untereinheit p40 von IL-12 aus Säugern, die IL-12-vermittelte Aktivität inhibieren.

9. Diagnostikum nach Anspruch 8, enthaltend die humane p40 Untereinheit von IL-12.

## Claims

1. The use of the natural or recombinant p40 subunit of mammalian IL-12, or parts or variants of the p40 subunit of mammalian IL-12 which inhibit IL-12-mediated activity, for producing a pharmaceutical for treating pathological states which are accompanied by a dysregulation of IL-12-mediated activities.

2. The use as claimed in claim 1, wherein the p40 subunit of IL-12 is of human origin.

3. The use as claimed in claim 1, wherein the pathological states are autoimmune diseases such as systemic lupus erythematodes, Wegner's syndrome or rheumatoid arthritis, bacterial or viral infections, or certain solid tumors or leukemia.

4. The use of the natural or recombinant p40 subunit of mammalian IL-12, or parts or variants of the p40 subunit of mammalian IL-12 which inhibit IL-12-mediated activity, for producing a diagnostic agent for detecting IL-12.

5. The use as claimed in claim 4, wherein the p40 subunit of IL-12 is of human origin.

6. A method of detecting the p40 subunit of mammalian IL-12, wherein a sample which is to be analyzed, and which contains the p40 subunit of mammalian IL-12, is removed, a defined quantity of IL-12 is added and the inhibitory activity of p40 on IL-12 is measured.

7. The method as claimed in claim 6, wherein the sample to be analyzed is derived from a human body fluid.

8. A diagnostic agent which comprises the natural or recombinant p40 subunit of mammalian IL-12, or parts or variants of the p40 subunit of mammalian IL-12 which inhibit IL-12-mediated activity.

9. A diagnostic agent as claimed in claim 8, which comprises the human p40 subunit of IL-12.

## Revendications

1. Utilisation de la sous-unité p40 naturelle ou recombinante de l'interleukine 12 (IL-12) de mammifères, ou de parties ou variantes de la sous-unité p40 d'IL-12 de mammifères, qui inhibent l'activité médiée par IL-12, pour la fabrication d'un médicament destiné au traitement d'états pathologiques qui apparaissent avec une dysrégulation d'activités médiées par I'IL-12.

2. Utilisation selon la revendication 1, caractérisée en ce que la sous-unité p40 d'IL-12 est d'origine humaine.

3. Utilisation selon la revendication 1, caractérisée en ce que, en ce qui concerne les états pathologiques, il s'agit de maladies auto-immunes, telles que le lupus érythémateux disséminé, le syndrome de Wegener ou la polyarthrite rhumatoïde, d'infections bactériennes ou virales ou de certaines tumeurs solides ou de leucémie.

4. Utilisation de la sous-unité p40 naturelle ou recombinante d'IL-12 de mammifères, ou de parties ou variantes de la sous-unité p40 d'IL-12 de mammifères, qui inhibent l'activité médiée par IL-12, pour la fabrication d'un agent de diagnostic pour la détection d'IL-12.

5. Utilisation selon la revendication 4, caractérisée en ce que la sous-unité p40 d'IL-12 est d'origine humaine.

6. Procédé pour la détection de la sous-unité p40 d'IL-12 de mammifères, caractérisé en ce que l'on prélève un échantillon à analyser qui contient la sous-unité p40 d'IL-12 de mammifères, on y ajoute une quantité définie d'IL-12 et on mesure l'activité inhibitrice de p40 sur IL-12.

7. Procédé selon la revendication 6, caractérisé en ce que l'échantillon à analyser provient d'un liquide corporel humain.

8. Agent de diagnostic, contenant la sous-unité p40 naturelle ou recombinante d'IL-12 de mammifères ou des parties ou variantes de la sous-unité p40 d'IL-12 de mammifères, qui inhibent l'activité médiée par IL-12.

9. Agent de diagnostic selon la revendication 8, contenant la sous-unité p40 humaine d'IL-12.
